(19) Europäisches Patentamt
European Patent Office
Office européen des brevets

(11) **EP 4 616 720 A1**

(12) **EUROPEAN PATENT APPLICATION**
published in accordance with Art. 153(4) EPC

(43) Date of publication:
**17.09.2025 Bulletin 2025/38**

(21) Application number: 24788984.3

(22) Date of filing: **09.04.2024**

(51) International Patent Classification (IPC):
**A23K 20/142** (2016.01)    **A23K 40/30** (2016.01)
**A23K 40/10** (2016.01)    **A23K 10/12** (2016.01)
**C12P 13/08** (2006.01)

(52) Cooperative Patent Classification (CPC):
**A23K 10/12; A23K 20/142; A23K 40/10;**
**A23K 40/30; C12P 13/08**

(86) International application number:
**PCT/KR2024/004690**

(87) International publication number:
**WO 2024/215042 (17.10.2024 Gazette 2024/42)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **13.04.2023 KR 20230048719**

(71) Applicant: **CJ CheilJedang Corporation**
**Seoul 04560 (KR)**

(72) Inventors:
• **SHIN, Ji Hyun**
  **Seoul 04560 (KR)**
• **LEE, Yong-Chan**
  **Seoul 04560 (KR)**
• **LEE, Su Jin**
  **Seoul 04560 (KR)**
• **KIM, Min Sup**
  **Seoul 04560 (KR)**
• **PARK, Sang Min**
  **Seoul 04560 (KR)**

(74) Representative: **Meissner Bolte Partnerschaft mbB**
**Patentanwälte Rechtsanwälte**
**Postfach 86 06 24**
**81633 München (DE)**

(54) **CORE-SHELL STRUCTURED LYSINE GRANULE**

(57)    The present disclosure relates to lysine granules having a core-shell structure, comprising: a core comprising lysine; and a shell comprising lysine hydrates, which is provided on the core surface, and a production method thereof. According to the present disclosure, a lysine hydrate shell is provided on the surface of the lysine granules, and since the lysine hydrate shell has low hygroscopicity, deliquescence, or agglomeration of lysine granules due to moisture can be reduced.

[FIG. 1]

**Description**

**[Technical Field]**

The present disclosure relates to lysine granules having a core-shell structure which can reduce moisture adsorption and deliquescence.

**[Background Art]**

In the conventional lysine production method, moisture absorption, deliquescence, and agglomeration of granules occur, when the added amount of anions such as hydrochloric acid and sulfuric acid is low, which makes distribution difficult and reduces marketability. This phenomenon occurs because when the number of anion molecules relative to lysine molecules is insufficient, the ratio of cationic lysine molecules to neutral lysine molecules increases, and the cationic lysine molecules have relatively strong hydrophilicity.

In this case, moisture absorption, deliquescence, and agglomeration may be alleviated or prevented when the added amount of hydrochloric acid and sulfuric acid is increased. However, hydrochloric acid and sulfuric acid have no nutritional value as feed, and if administered excessively, they may have a negative effect on animal health. In addition, when additional hydrochloric acid or sulfuric acid is added, there is a disadvantage that the material cost of the product increases as much as the cost of the added chemicals. Further, various environmental pollutants may be generated when producing hydrochloric acid or sulfuric acid, which may be problematic.

In one example, lysine powder products are produced in the form of lysine hydrochloride by adding hydrochloric acid in the same molar ratio as lysine, followed by a crystallization process. In lysine hydrochloride crystals, the lysine content is theoretically 80%, but considering that the quality standard for the content of commonly distributed lysine hydrochloride is 98% or more, the actual lysine content is about 79%. In another example, lysine granules are mainly produced containing sulfuric acid. However, because sulfate ions have fewer anions relative to hydrochloric acid ions for the same mass, the content of lysine granule products is lower than that of lysine powder products. Commercial lysine granules that are commonly distributed are in the form of lysine sulfate and have a lysine content of 56 to 64%. If the products are produced by increasing the content by reducing the ratio of sulfuric acid, the marketability may be reduced due to moisture absorption, deliquescence, and agglomeration.

**[Prior Art Document]**

**[Patent Document]**

(Patent Document 1) US 11104925 B2

**[Disclosure]**

**[Technical Problem]**

It is one object of the present disclosure to provide lysine granules having a core-shell structure, in which lysine hydrates are provided on the surface, thereby significantly reducing moisture absorption, deliquescence and agglomeration.

**[Technical Solution]**

The present disclosure relates to lysine granules having a core-shell structure, which have a shell comprising lysine hydrates, and a hydrate shell is formed on the core surface of lysine, and since the lysine hydrate shell has low hygroscopicity, moisture absorption of lysine granules, which are the final products, and the resulting deliquescence and agglomeration can be significantly reduced.

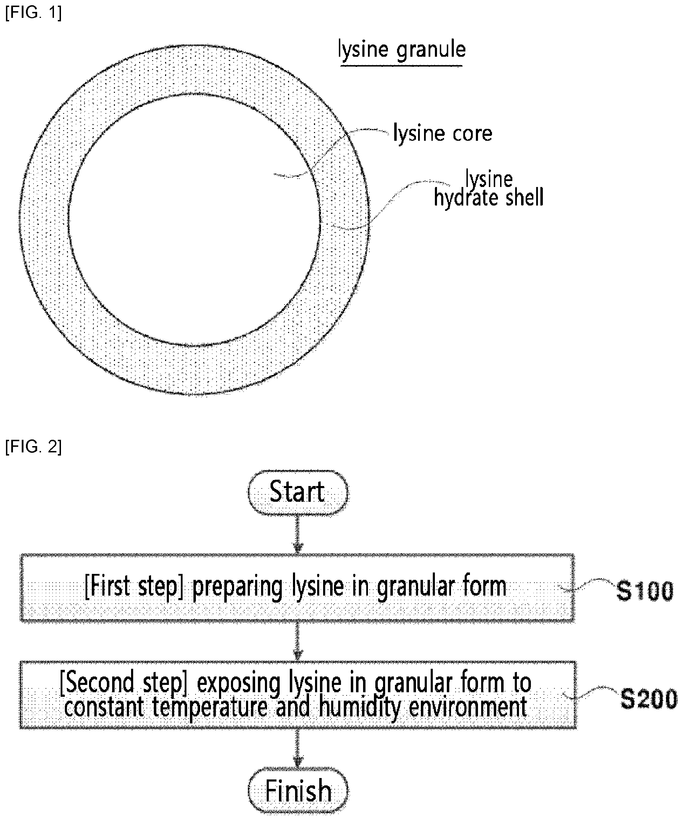

FIG. 1 is a cross-sectional view of lysine granules according to the present disclosure.

The lysine granules according to the present disclosure are provided in a core-shell structure, comprising a lysine core comprising lysine; and a lysine hydrate shell comprising lysine hydrates, which is provided on the core surface.

The lysine granules comprise L-lysine and are provided in granular form. The "granules" are macroscopic particles, which are larger-sized permanent aggregates composed of small particles such as powder, and may be particles with an average particle diameter of 50 $\mu$m to 5 mm, 75 $\mu$m to 4 mm, or 100 $\mu$m to 3 mm.

The lysine granules are shown in the drawing as having a spherical shape, but this is only an exemplary shape for ease of explanation. As described above, granules are aggregates composed of small particles and can have various shapes. For example, granules can have various shapes, such as irregular shapes, tetrahedrons, hexahedrons, *etc.*

The lysine granules comprise a lysine core and a lysine hydrate shell.

The lysine core may be a region comprising lysine and no or substantially no lysine hydrate. Substantially not comprising lysine hydrate means that the content of lysine hydrate is insignificant that the effect of reducing moisture absorption due to lysine hydrate does not appear.

The lysine core may be provided in the central portion of the lysine granule. However, providing the lysine core in the central portion does not necessarily mean that the center of the lysine granule and the lysine core coincide. For example, when the lysine granules have an irregular shape, it is difficult to find out where the center of the lysine granule is. Therefore, providing the lysine core in the central portion of the lysine granule may mean that the lysine core is provided in the remaining region of the lysine granule excluding the lysine hydrate shell portion.

The lysine hydrate shell is provided on the surface of the lysine core.

The lysine granules below may have a hydration rate according to Equation 1 of 11% to 23%, specifically 12% to 23%, 13% to 23%, 13% to 21%, 14% to 23%, 15% to 23%, 15% to 21%, 16% to 23%, 16% to 21%, 18% to 23%, 18% to 21%, or 19% to 21%.

[Equation 1]

$$Hydration\ rate\ (\%) = \frac{(W_0 - W_1)}{W_0} \times 100$$

In Equation 1 above, $W_1$ represents the lysine content (wt%) in the granules after hydration of the lysine granules, and $W_0$ represents the lysine content (wt%) in the granules before hydration of the lysine granules.

The moisture content in the lysine granules may be in the range of 10 wt% to 35 wt%, 12 wt% to 28 wt%, 12 wt% to 25 wt%, 13 wt% to 22 wt%, 14 wt% to 22 wt%, 14 wt% to 21 wt%, 16 wt% to 22 wt%, or 18 wt% to 22wt%, based on the total weight of the lysine granules.

The moisture content in the lysine granules may be in the range of 14 wt% to 35 wt%, 14 wt% to 30 wt%, 14 wt% to 29 wt%, 15 wt% to 29 wt%, 16 wt% to 27 wt%, 18 wt% to 27 wt%, 20 wt% to 27 wt%, or 22 wt% to 27wt%, based on the weight of lysine.

The lysine hydrate shell is provided to surround the lysine core on the inside, protecting the lysine core from the outside. That is, the lysine hydrate shell is provided on the surface of the lysine core, so that the lysine core is not exposed to the outside. Accordingly, even if lysine granules are exposed to a high humidity environment, the low hygroscopic lysine hydrate shell protects the highly hygroscopic lysine core.

The lysine hydrate shell comprises lysine hydrates. The lysine hydrate may be a molecule in which one or more equivalent(s) of water molecules can be bound to one equivalent of L-lysine molecules. In some cases, the lysine hydrate may be a molecule in which two or more equivalents of water molecules are bound to one equivalent of L-lysine molecules. L-lysine is a polar molecule in which positive and negative charges coexist within the molecule and has high affinity for water molecules. Accordingly, in general, lysine can absorb water molecules present in the atmosphere. However, when lysine simply absorbs water molecules present in the air, lysine monohydrate is formed in which one equivalent of water molecules is bound to one equivalent of L-lysine molecules, instead of an excess of water molecules being bound to the L-lysine molecules. In the case of lysine monohydrate, it is still possible that it may bind with water molecules, which may lead to additional moisture absorption, resulting in deliquescence and agglomeration of lysine granules. In contrast, the lysine hydrate having two or more equivalents of water molecules bound thereto can maintain its shape without additional moisture absorption in the atmosphere. Therefore, the lysine core on the inside the lysine granule may be protected by providing the lysine hydrate bound to two or more equivalents of water molecules in the lysine hydrate shell.

The above-described lysine hydrate comprised in the lysine hydrate shell has a high degree of crystallinity, and thus it is unlikely to form a bridge between lysine molecules. Accordingly, solidification and agglomeration due to bridging between lysine molecules may be prevented.

The content of lysine in lysine granules may be 64 wt% to 86 wt% based on the total weight of lysine granules. In some cases, the content of lysine in lysine granules may be 64 wt% to 80 wt%, 64 wt% to 75 wt%, 64 wt% to 70 wt%, 70 wt% to 86 wt%, 75 wt% to 86 wt%, 80 wt% to 86 wt%, 70 wt% to 80 wt% or 75 wt% to 80 wt% based on the total weight of lysine granules. When the lysine content in the lysine granules is above a certain amount as described above, the content of lysine hydrate provided in the lysine hydrate shell may be sufficient, and the lysine hydrate is provided in a form in which two or more water molecules are bound, and thus may have an excellent core protection effect by the lysine hydrate shell. Therefore, in order to increase the lysine content in lysine granules for commercial use and also prevent moisture absorption, deliquescence and agglomeration according to the present disclosure, the lysine content may be adjusted to the above-mentioned range.

Lysine granules may be provided in free base form.

Lysine granules may be provided without lysine hydrochloride or lysine sulfate. Conventionally, hydrochloric acid and/or sulfuric acid were added during the production of lysine granules so that lysine hydrochloride or lysine sulfate could be included in the lysine granules. However, in this case, there was a problem that the lysine content in the lysine granules was reduced. Considering that the quality standard for the content of lysine hydrochloride is 98% or more, the actual lysine content is about 79%. **In** another example, lysine granules are mainly produced with sulfuric acid contained therein. However, because sulfate ions have fewer anions relative to hydrochloric acid ions for the same mass, the content of lysine granule products is lower than that of lysine powder products. Commercial lysine granules that are commonly distributed are in the form of lysine sulfate with a lysine content of 56 to 64%. **In** comparison, according to the present disclosure, the lysine content in lysine granules may be increased and at the same time, deliquescence and agglomeration due to moisture absorption may be prevented when a lysine hydrate shell comprising lysine hydrates is provided only on the surface with exclusion of lysine hydrochloride and lysine sulfate in lysine granules.

Next, FIG. 2 is a flow chart showing the method for producing lysine granules according to the present disclosure.

With reference to FIG. 2, the method according to the present disclosure comprises a first step (S100) of producing lysine in granular form; and a second step (S200) of exposing the lysine in granular form to a constant temperature and humidity device (also known as"Thermo-Hygrostat") to provide a shell comprising lysine hydrates on the surface of the granular lysine, thereby producing lysine granules having a core-shell structure.

**The** detailed descriptions regarding the lysine granules having a core-shell structure prepared according to the production method of the present disclosure are the same as those discussed above. Therefore, hereinafter, the characteristics of the production method will be discussed to avoid duplication of content.

In order to carry out the method for preparing lysine of the present disclosure, a first step (S100) of preparing lysine in granular form is performed.

In the first step (S100), granular lysine may be prepared by purchasing commercially available granular lysine. However, in some cases, L-lysine in granular form may be prepared by preparing a fermentation liquid comprising L-lysine and then drying the fermentation liquid.

In this case, as used herein, the "fermented product" may refer to a product resulting from enzymatic or metabolic decomposition of organic substances using a microorganism. For example, the fermented product may include the culture itself obtained by culturing a microorganism in a culture medium, or a concentrate, dried product, or freeze-dried product of the culture obtained by removing the strain therefrom. In addition, in this case, the fermentation liquid may include the entire fermented product comprising amino acids, or may be one in which impurities have been removed from the fermented product comprising amino acids.

The "L-lysine-producing strain" used in the culturing step includes all wild-type microorganisms, or naturally or artificially genetically modified microorganisms, and may be a microorganism in which a particular mechanism is weakened or enhanced due to insertion of a foreign gene, or enhancement or inactivation of the activity of an endogenous gene, *etc.,* and may be a microorganism having genetic modification to produce a desired protein or amino acid.

The L-lysine-producing strain of the present disclosure may be a microorganism naturally having an L-lysine-producing ability, or a microorganism, in which an L-lysine-producing ability has been imparted to a parent strain having no L-lysine-producing ability, but is not limited thereto. Specifically, as used herein, the microorganism for producing L-lysine or a desired product, or the microorganism having an L-lysine or desired product-producing ability may be a microorganism in which some genes in the biosynthesis pathway of the desired protein or desired product are enhanced or weakened, or some genes in the degradation pathway of the desired protein or desired product are enhanced or weakened. The "enhancement" or "increase" in the L-lysine-producing ability of the microorganism of the present disclosure may mean that the L-lysine-producing ability of the microorganism of the present disclosure is enhanced compared to that of a microorganism other than the microorganism of the present disclosure, a parent strain, or a non-modified microorganism. In one example, the microorganism of the present disclosure may have an enhanced L-lysine-producing ability by about 1% or more, 10% or more, 100% or more, 200% or more, 500% or more, 1000% or more, 1100% or more, 1200% or more, 1300% or more, or about 1.01 times or more, 2 times or more, 5 times or more, 10 times or more, 11 times or more, 12 times or more, or 13 times or more compared to the L-lysine-producing ability of other microorganisms, but is not limited thereto. As used herein, the term "about" refers to a range including all of ±0.5, ±0.4, ±0.3, ±0.2, ±0.1, *etc.,* and it includes all of the values equivalent to those which come immediately after the term "about" or those in a similar range, but is not limited thereto.

The above-described microorganism used in the culturing step may be at least one selected from the group consisting of *Candida famata,* which is a yeast, *Eremothecium ashbyii* and *Ashbyagossypii,* which are ascomycetes, *Bacillus subtilis* and microorganisms belonging to the genus *Corynebacterium,* which are bacteria.

When the microorganism used in the culturing step is a microorganism belonging to the genus *Corynebacterium,* the microorganism may specifically be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium crenatum,* or *Corynebacterium flavescens,* more specifically, *Corynebacterium glutamicum,* but is not limited thereto.

The microorganism of the genus *Corynebacterium,* in particular, *Corynebacterium glutamicum* is a Gram-positive microorganism that is widely used to produce L-amino acids and other beneficial substances. In order to produce L-amino acids and other beneficial substances, various research has been performed to develop microorganisms with high-efficiency production and technologies for fermentation processes. For example, target-specific approaches, such as a method of increasing expression of a gene encoding an enzyme involved in biosynthesis of L-lysine, *etc.,* or a method of removing a gene unnecessary for biosynthesis, have been widely used. In the present disclosure, a fermentation liquid comprising amino acids can be prepared using a strain of the genus *Corynebacterium.*

After preparing the fermentation liquid, the step of separating L-lysine from the fermentation liquid and granulating the separated L-lysine may be additionally performed.

For example, the prepared fermentation liquid may be concentrated, and the L-lysine wet crystals produced during the concentration process may be separated. In this process, L-lysine may be precipitated in the form of crystals by removing moisture from the fermentation liquid. The concentration may be carried out in various ways. The concentration may be carried out using a conventional concentrator (e.g., paddle dryer, slurry drying equipment, reduced pressure concentrator, forced circulation concentrator, thin film concentrator, or rotary concentrator, *etc.)* according to the appropriate selection by those skilled in the art.

In addition, a solid-liquid separator such as a reduced-pressure membrane filtration device, a pressurized membrane filtration device, a centrifugal separator, *etc.* may be used to separate the L-lysine wet crystals precipitated by concentration from the mother liquor, but is not limited thereto.

The mother liquor remaining after the separation of L-lysine wet crystals may be used again for concentration. Accordingly, L-lysine that did not form wet crystals or L-lysine that precipitated as crystals of a certain size or less, which was not separated in the separation step of L-lysine wet crystals and remained in the mother liquor may be recycled. Additionally, after circulation of the mother liquor, an additional process such as heating the fermentation liquid may be performed, if necessary, to dissolve the L-lysine precipitated in the form of fine crystals again in the fermentation liquid.

Subsequently, a step of preparing L-lysine mixed granules may be performed by mixing the separated L-lysine wet crystals with a seed. The seed used in the above step, which is also called a crystal of a seed or a seed crystal, may refer to a material used as a catalyst for crystallization or granulation of a liquid. Specifically, the seed in the present disclosure may refer to a crystal of an L-amino acid, for example, a crystal of L-lysine of the same type as the L-lysine comprised in the fermentation concentrate to be granulated, but is not limited thereto. When the seed and a fermentation liquid meet, the solid components present in the fermentation liquid are combined with the seed to form an aggregation, thereby forming granules. The seed used in this step may have an average particle size of 150 to 300 μm. Specifically, a seed having an average particle size of 150 to 250 μm, 200 to 300 μm, or 200 to 250 μm may be used, but is not limited thereto. The particle size of the seed used may ultimately affect the productivity of the granule preparation according to the present disclosure, and thus can be appropriately selected by those skilled in the art considering the desired moisture content, *etc.*

In the above step, the L-lysine mixed granules may be prepared using a mixed granulator. The granules may be obtained via the mixed granulator by injecting the seed at a constant rate through a feeder into the mixed granulator and simultaneously supplying the previously obtained amino acid wet crystals. Herein, the "granules" are macroscopic particles, which are larger-sized permanent aggregates composed of small particles such as powder, and may be particles with an average particle diameter of 50 μm to 5 mm, 75 μm to 4 mm, or 100 μm to 3 mm.

Additionally, in the step of preparing the lysine in granular form, removal of strains, desalting, and concentration of the fermentation liquid may be performed. Removal of strains may be performed by various methods such as filtration, centrifugation, *etc.* Additionally, in the step of preparing the lysine in granular form, a desalting process may be performed. The desalting process may be performed to remove ionic impurities other than the fermentation product to be produced. The desalting process may be performed by various methods, such as ion exchange resins, continuous chromatography, *etc.* The concentration process may increase the concentration of fermentation products in the process liquid and enable to more easily obtain the fermentation products in the subsequent drying process. There are no limitations of performing the concentration process, and various methods such as rotary concentrator, *etc.,* may be used. The order of filtration, decolorization, strain removal, and desalting described above may be varied as needed. The filtration, decolorization, and desalting may be performed after removal of strains, or the strains may be removed after filtration, decolorization, and desalting.

Subsequently, a second step (S200) of exposing the lysine in granular form to a constant temperature and humidity device to provide a shell comprising lysine hydrates on the surface of the granular lysine is performed.

The second step (S200) is a step of providing a lysine hydrate shell by forming lysine hydrate on the surface of the prepared granular lysine.

In particular, as discussed above, L-lysine has excellent affinity for water molecules because it contains a polar functional group within the molecule. Therefore, even when exposed to a general atmospheric environment, L-lysine can react with water molecules in the air and turn into L-lysine hydrate. However, in this case, L-lysine monohydrate is mostly formed. The L-lysine monohydrate can be additionally hydrated, thus it has a poor moisture absorption prevention effect. Therefore, even if L-lysine monohydrate is provided on the surface of L-lysine granules, the moisture absorption prevention effect to be achieved in the present disclosure may not be obtained.

Accordingly, in the second step (S200), the granular lysine is exposed to a constant temperature and humidity environment with a specific humidity and temperature, thereby allowing a sufficient hydration reaction to occur in the surface layer of the granular lysine. Specifically, the second step (S200) may be performed to uniformly provide lysine hydrate in which two or more equivalents of water molecules are bound to one equivalent of L-lysine molecules on the surface layer of the granular lysine.

In the second step (S200), the constant temperature and humidity environment may be implemented in the form of a chamber capable of controlling temperature and humidity. However, in addition to the above, the constant temperature and humidity environment may be implemented in various forms. For example, a constant temperature and humidity environment may be implemented in the form of transporting granular lysine into a pipe where temperature and humidity can be controlled.

The constant temperature and humidity environment implemented in the second step (S200) may be an environment in which the relative humidity is 25% to 60% at a temperature of 30°C. Additionally, if necessary, the constant temperature and humidity environment may be implemented by gradually increasing the humidity. In this case, as the humidity in the environment to which the granular lysine is exposed slowly increases, L-lysine may be uniformly converted into lysine hydrate in which two or more equivalents of water molecules are bound to one equivalent of L-lysine molecules. Therefore, the lysine hydrate may be provided more uniformly throughout the entire surface area compared to when lysine is immediately exposed to a high humidity environment.

Specifically, the second step (S200) may be performed by exposing the lysine in granular form to an environment with a relative humidity of 40% and then increasing the relative humidity of 50% to 60% at a rate of 0.2% per hour. In this case, the lysine hydrate shell may be formed more quickly. As such, in the second step (S200) of the present disclosure, the constant temperature and humidity environment means an environment in which the relative humidity and temperature may be adjusted and maintained at a specific value according to the user's needs, and does not mean an environment in which the temperature and humidity do not change and remain constant throughout the second step (S200).

The second step (S200) may further comprise drying the lysine granules after forming the lysine hydrate shell. Drying of lysine granules may be performed in various ways, such as a fluid-bed dryer, a tray, an oven, *etc.*

The above-described method for preparing lysine granules may be performed without the addition of anionic substances such as hydrochloric acid and sulfuric acid, or the amount thereof may be greatly reduced. Accordingly, the prepared lysine granules may comprise no lysine anion salts, including lysine sulfate and lysine hydrochloride, or the content thereof may be very low. Therefore, even if the prepared lysine granules are used as animal feed, adverse effects on animal nutrition due to lysine anionic salts may be prevented, and preparation process costs and environmental pollutant emissions may be reduced.

Each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

**[Advantageous Effects]**

According to the present disclosure, a lysine hydrate shell is provided on the surface of the lysine core, and since the lysine hydrate shell has low hygroscopicity, it is possible to reduce deliquescence or agglomeration of lysine granules due to moisture.

In addition, according to the present disclosure, granules with a high lysine content can be prepared because the adverse effects caused by moisture absorption can be prevented without lysine hydrochloride or lysine sulfate or while drastically reducing the contents thereof.

**[Brief Description of Drawings]**

FIG. 1 is a cross-sectional view of lysine granules according to the present disclosure.
FIG. 2 is a flow chart of the method for preparing lysine granules according to the present disclosure.
FIGS. 3a to 3c are images showing the surface of lysine granules according to the humidity in a constant temperature and humidity chamber.
FIGS. 4a to 4c are images showing the appearance of lysine granules according to the exposure environment in a constant temperature and humidity chamber.

**[Detailed Description of Preferred Embodiments]**

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

In the above, the lysine granules and the method for producing lysine granules according to one aspect of the present disclosure have been examined. Hereinafter, the beneficial effects mentioned in the present disclosure will be discussed by the experimental results of Examples and Comparative Examples.

**Experimental Example 1. Surface Analysis of Lysine Granules according to Relative Humidity**

In the Experimental Example, the changes in the surface of lysine granules were observed by changing the relative humidity in a constant temperature and humidity environment for commercially produced lysine granules.

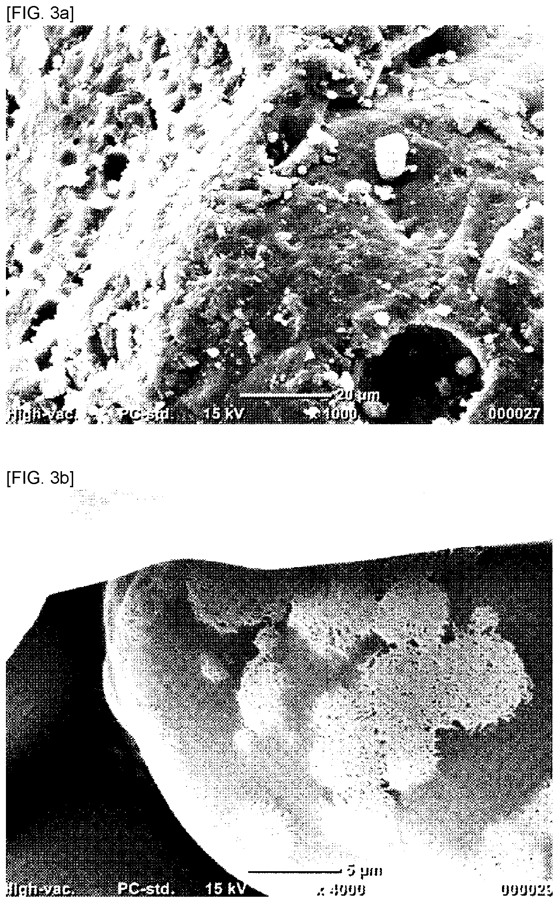

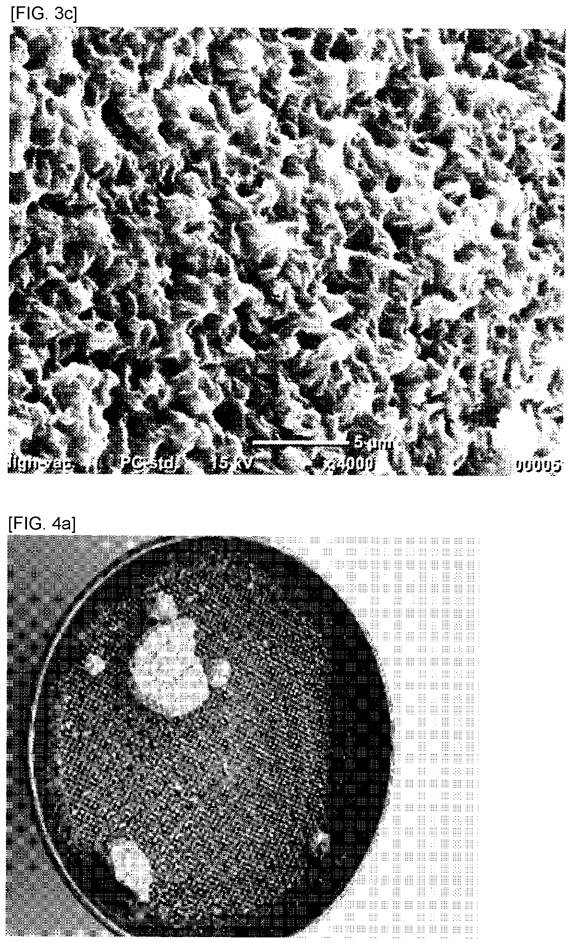

FIGS. 3a to 3c are images showing the surface of lysine granules according to the humidity in a constant temperature and humidity chamber.

**Example 1. Preparation of 99% lysine granules (free base)**

An aqueous amino acid solution was prepared by purifying a fermentation liquid comprising L-lysine. For the preparation of the fermentation liquid, a strain of *Corynebacterium* sp. capable of producing L-lysine was seed-cultured in 25 mL of a seed medium (pH 7.0) under 30°C and 200 rpm conditions for 20 hours. The seed medium consisted of 20 g of glucose, 10 g of peptone, 5 g of yeast extract, 1.5 g of urea, 4 g of $KH_2PO_4$, 8 g of $K_2HPO_4$, 0.5 g of $MgSO_4·7H_2O$, 100 μg of biotin, 1 mg of thiamine HCl, 2 mg of calcium-pantothenic acid, and 2 mg of nicotinamide, based on 1 L of distilled water. The seed bacteria obtained through seed culture were inoculated into a production medium (pH 7.0) at 4% (v/v) and cultured at 30°C with sufficient aeration and stirring until all the added glucose was consumed, and thereby a final fermentation liquid was obtained. The production medium consisted of 100 g of glucose, 40 g of $(NH_4)_2SO_4$, 2.5 g of soy protein, 5 g of corn steep solids, 3 g of urea, 1 g of $KH_2PO_4$, 0.5 g of $MgSO_4·7H_2O$, 100 μg of biotin, 1 mg of thiamine HCl, 2 mg of calcium-pantothenic acid, 3 mg of nicotinamide, and 30 g of $CaCO_3$, based on 1 L of distilled water. After completion of the culture, the concentration of the L-lysine in the fermentation liquid was analyzed using HPLC (Waters Corporation, 2478). The microorganisms in the fermentation liquid were removed using a membrane with a pore size of 0.1 μm. The fermentation liquid from which microorganisms were removed was passed through a cation exchange resin tower to allow the L-lysine in the fermentation liquid to be adsorbed to the resin tower to thereby separate L-lysine from other impurities. The adsorbed L-lysine was recovered by desorbing it from the resin tower using about 2 N ammonia water, and then concentrated by heating in a vacuum state to prepare an aqueous L-lysine solution, and the solid materials in the aqueous solution were controlled around 50% during the concentration procedure. After the concentration step, the concentration of the L-lysine aqueous solution was 560 g/L, and the pH was 10.2, specific gravity was 1.13, and purity was 99 wt%.

The prepared aqueous solution was granulated by injecting it into a fluidized bed granulator at a rate of 15 mL/min and then spraying it into a granulator at 80°C at a nozzle pressure of 1.2 kg/cm$^2$. The prepared granules were selected to have a size of about 0.425 mm to about 2.0 mm through sieving.

The 99% lysine granules (free base) were shown to contain 99 wt% of lysine, 0.4 wt% of ions, 0.1 wt% of other amino acids, and 0.5 wt% of moisture.

**Example 2. Preparation of 74% lysine granules (free base)**

An aqueous amino acid solution was prepared by purifying a fermentation liquid comprising L-lysine. For the preparation of the fermentation liquid, a strain of *Corynebacterium* sp. capable of producing L-lysine was seed-cultured in 25 mL of a seed medium (pH 7.0) under 30°C and 200 rpm conditions for 20 hours. The seed medium consisted of 20 g of glucose, 10 g of peptone, 5 g of yeast extract, 1.5 g of urea, 4 g of $KH_2PO_4$, 8 g of $K_2HPO_4$, 0.5 g of $MgSO_4·7H_2O$, 100 μg of biotin, 1 mg of thiamine HCl, 2 mg of calcium-pantothenic acid, and 2 mg of nicotinamide, based on 1 L of distilled water. The seed bacteria obtained through seed culture were inoculated into a production medium (pH 7.0) at 4% (v/v) and cultured at 30°C with sufficient aeration and stirring until all the added glucose was consumed, and thereby a final fermentation liquid was obtained. The production medium consisted of 100 g of glucose, 40 g of $(NH_4)_2SO_4$, 2.5 g of soy protein, 5 g of corn steep solids, 3 g of urea, 1 g of $KH_2PO_4$, 0.5 g of $MgSO_4·7H_2O$, 100 μg of biotin, 1 mg of thiamine HCl, 2 mg of calcium-pantothenic acid, 3 mg of nicotinamide, and 30 g of $CaCO_3$, based on 1 L of distilled water. After completion of the culture, the concentration of the L-lysine in the fermentation liquid was analyzed using HPLC (Waters Corporation, 2478). The microorganisms in the fermentation liquid were removed using a membrane with a pore size of 0.1 μm. The

fermentation liquid from which microorganisms were removed was concentrated by heating in a vacuum state to prepare an aqueous L-lysine solution, and the solid materials in the aqueous solution were controlled around 50% during the concentration procedure. After the concentration step, the concentration of the L-lysine aqueous solution was 418 g/L, and the pH was 8.5, specific gravity was 1.13, and purity was 74 wt%.

The prepared aqueous solution was granulated by injecting it into a fluidized bed granulator at a rate of 15 mL/min and then spraying it into a granulator at 80°C at a nozzle pressure of 1.2 kg/cm$^2$. The prepared granules were selected to have a size of about 0.425 mm to about 2.0 mm through sieving.

The 74% lysine granules (free base) were shown to contain 74 wt% of lysine, 12 wt% of ions, 0.8 wt% of other amino acids, 0.5 wt% of moisture, and about 13 wt% of others.

**Example 3. Increment of relative humidity up to 60% at a rate of 0.2% per hour from 40% for 99% lysine granules**

300 g of the 99% lysine granules prepared in Example 1 above were placed into a plastic dish and into a constant temperature and humidity chamber at a temperature of 30°C and a relative humidity of 40%. Thereafter, the relative humidity was increased to 60% at a rate of 0.2% per hour. FIGS. 3a to 3c show scanning electron micrographs of the surface of lysine granules before placing them into the constant temperature and humidity chamber, and at a relative humidity of 50% and a relative humidity of 60%, respectively. It was confirmed that lysine hydrate crystals were coated on the entire surface of lysine granules over time. Thereafter, drying was performed in an oven dryer at a temperature of 70°C for 24 hours to obtain lysine granules whose surface was coated with lysine hydrate crystals.

The surface-coated lysine granules were shown to contain 79 wt% of lysine, 0.3 wt% of ions, 0.1 wt% of other amino acids, and about 21 wt% of moisture.

**Example 4. Increment of relative humidity up to 60% at a rate of 0.2% per hour from 40% for 74% lysine granules**

300 g of the 74% lysine granules prepared in Example 2 above were placed in a plastic dish and into a constant temperature and humidity chamber at a temperature of 30°C and a relative humidity of 40%. Thereafter, the relative humidity was increased to 60% at a rate of 0.2% per hour. Then, drying was performed in an oven dryer at a temperature of 70°C for 24 hours to obtain lysine granules whose surface was coated with lysine hydrate crystals.

The surface-coated lysine granules were shown to contain 64 wt% of lysine, 10 wt% of ions, 1 wt% of other amino acids, and about 11.4 wt% of other components.

**Example 5. Increment of relative humidity up to 50% at a rate of 0.2% per hour from 40% for 99% lysine granules**

300 g of the 99% lysine granules prepared in Example 1 above were placed in a plastic dish and into a constant temperature and humidity chamber at a temperature of 30°C and a relative humidity of 40%. Thereafter, the relative humidity was increased to 50% at a rate of 0.2% per hour. Then, drying was performed in an oven dryer at a temperature of 70°C for 24 hours to obtain lysine granules whose surface was coated with lysine hydrate crystals.

The surface-coated lysine granules were shown to contain 86 wt% of lysine, 0.4 wt% of ions, 0.1 wt% of other amino acids, and about 14 wt% of moisture.

As a result of observing the surface of the lysine granules prepared according to Examples 3 to 5 described above as shown in FIGS. 3a to 3c, it was confirmed that the surface of the lysine granules changed after exposing the prepared lysine granules to a constant temperature and humidity environment. This suggests that lysine hydrates were provided on the surface of lysine granules as described above.

Hereinafter, it was confirmed whether changes in physical properties such as moisture absorption rate and agglomeration rate appeared when lysine hydrate was provided on the surface of lysine granules as described above.

**Experimental Example 2. Analysis of the Content, Moisture Absorption Rate, Agglomeration Rate, and Results After Treatment in Constant Temperature and Humidity Chamber and Shaker of Lysine Granule**

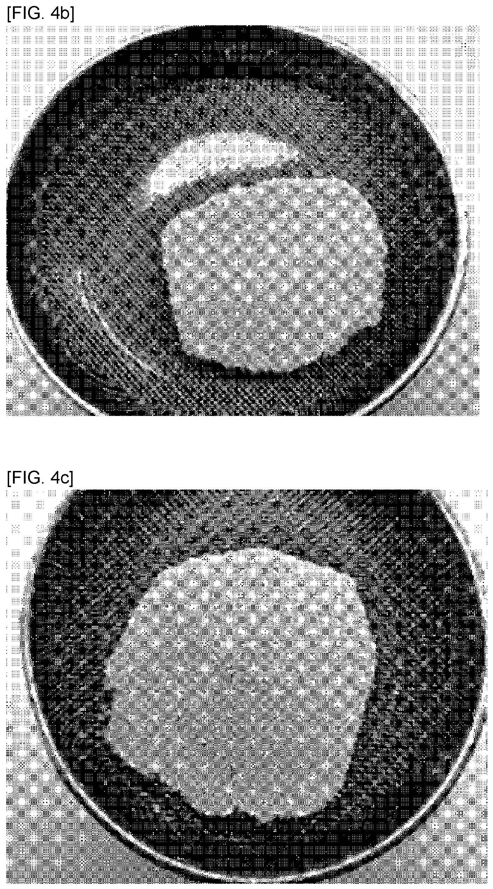

The content, moisture absorption rate, agglomeration rate, and the results after treatment in a constant temperature and humidity chamber and shaker of lysine granules coated with lysine crystals on the surface obtained in Examples 3 to 5 are shown in FIGS. 4a to 4c. The purpose of the present disclosure was to reduce impurities such as anions, improve content, and produce granules with low solidification. Therefore, moisture absorption rate and agglomeration rate were used as indicators to determine solidification. As the moisture absorption rate and agglomeration rate increased, the hygroscopicity and solidification properties of the products became significant, which made the handling more difficult.

In the following analysis, the moisture absorption rate was calculated in the following manner.

Moisture Absorption Rate (%) = ([Sample mass after treatment in the constant temperature and humidity chamber] - [Sample mass before treatment in the constant temperature and humidity chamber])/[Sample mass before treatment in the constant temperature and humidity chamber] x 100

In addition, in order to evaluate the solidification of the granules, 140 g of moisture-absorbed granules were collected, placed in a 14cm x 14cm plastic bag, and then pressed for 10 minutes under a pressure of 7 bar using a press. The pressurized sample was transferred to a 1.7 mm sieve, placed on a sieve shaker, and shaken at a frequency of 50 Hz for 5 minutes. The solidification of the products was quantitatively expressed by the agglomeration rate, which can be calculated using the change in mass of the sample before and after treatment in the sieve shaker.

Additionally, for comparison purposes, the 99% lysine granules (free base) of Example 1, the 74% lysine granules (free base) of Example 2, and the 79% lysine hydrochloride powder (CJ CheilJedang, Korea) and the 64% lysine sulfate granules (CJ CheilJedang, Korea), which are commercially distributed, were analyzed using the same method.

FIGS. 4a to 4c are images after evaluating the solidification properties of the granules, showing the results for the granules of Examples 3 to 5, respectively.

The granules coated with lysine crystals on the surface prepared in Example 1 by the method proposed in the present disclosure were solidified at a level similar to that of lysine hydrochloride when produced at a content (79%) similar to that of the lysine hydrochloride powder product. Furthermore, the lysine granules have the advantage of reducing adverse effects on animal nutrition, reducing material costs of the product, and reducing environmental pollutant emissions by eliminating or reducing the use of anionic substances such as hydrochloric acid and sulfuric acid.

The granules coated with lysine crystals on the surface prepared in Example 2 by the method proposed in the present disclosure were less solidified than lysine sulfate granules when produced at a content (64%) similar to that of lysine sulfate granules. In addition, this has the advantage of eliminating or reducing the use of anionic substances such as hydrochloric acid and sulfuric acid, as in Example 1.

The granules coated with lysine crystals on the surface prepared in Example 3 by the method proposed in the present disclosure were solidified at a level similar to that of lysine sulfate granules when produced at a slightly higher content (86%) than the lysine hydrochloride powder product. This also has the advantage of eliminating or reducing the use of anionic substances such as hydrochloric acid and sulfuric acid, as in Example 1.

[Table 1]

| Item | Example 3 | Example 4 | Example 5 | Example 1 | Example 2 | Lysine Hydrochloride | Lysine Sulfate |
|---|---|---|---|---|---|---|---|
| Content (%) | 79 | 64 | 86 | 99 | 74 | 79 | 64 |
| Moisture Absorption Rate (%) | 5 | 8 | 12 | 42 | 30 | 5 | 6 |
| Agglomeration Rate (%) | 14 | 65 | 71 | 100 | 100 | 15 | 78 |

As can be seen in Table 1 above, the lysine granule product of Example 1 and lysine granule product of Example 2, which do not contain a lysine hydrate shell on the surface, have a high lysine content, but the moisture absorption and agglomeration rates were found to be very high. Therefore, it can be implied that the quality of these products can be significantly reduced in a high humidity environment.

In contrast, the lysine hydrochloride powder product and the lysine sulfate granule product had relatively low moisture absorption and agglomeration rates, but the lysine content was decreased. In particular, in the case of the lysine hydrochloride powder product, although the lysine content was somewhat excellent, and the moisture absorption and agglomeration rates were also good, it was provided in powder form and the agglomeration rate was low, making it difficult to further form into granules. The powder form has the disadvantage of being more difficult to handle and carry out subsequent processing compared to the granule form.

Accordingly, it was confirmed that the lysine granules according to the present disclosure could increase the lysine content while decreasing the moisture absorption and agglomeration rates by providing the shell containing lysine hydrate. In addition, when the shell containing lysine hydrate was provided on the surface of lysine granules, the moisture absorption rate was significantly lowered as described above, and thus it was found that once the lysine hydrate layer (shell layer) was formed to a certain depth with respect to the lysine granules, it was difficult to provide lysine hydrate inside the lysine granules any longer.

Therefore, through this experiment, it was confirmed that lysine granules having a core-shell structure can be prepared by exposing the lysine granules to specific conditions of humidity and temperature.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

**Claims**

1.  A lysine granule having a core-shell structure, comprising:

    a core comprising lysine; and
    a shell comprising lysine hydrates, which is provided on the core surface.

2.  The lysine granules of claim 1, wherein the lysine content in the lysine granules is 64 wt% to 86 wt%.

3.  The lysine granules of claim 1, wherein the lysine granules are in the form of a free base.

4.  The lysine granules of claim 1, wherein the lysine granules have a hydration rate of 11% to 23% in accordance with Equation 1 below:

    [Equation 1]

    $$Hydration\ rate\ (\%) = \frac{(W_0 - W_1)}{W_0} \times 100$$

    wherein in Equation 1 above, $W_1$ represents the lysine content (wt%) in the granules after hydration of the lysine granules, and $W_0$ represents the lysine content (wt%) in the granules before hydration of the lysine granules.

5.  A method for producing lysine granules, comprising:

    a first step of preparing lysine in granular form; and
    a second step of exposing the lysine in granular form to a constant temperature and humidity device to provide a shell comprising lysine hydrates on the surface of the granular lysine, thereby producing lysine granules having a core-shell structure.

6.  The method of claim 5, wherein, in the second step, the lysine in granular form is exposed to an environment with a relative humidity of 25% to 60%.

7.  The method of claim 6, wherein, in the second step, the lysine in granular form is exposed to an environment with a relative humidity of 40%, and then the relative humidity is increased to 50% to 60% at a rate of 0.2% per hour.

8.  The method of claim 5, wherein the lysine in granular form is provided by preparing a fermentation liquid comprising lysine and then drying the fermentation liquid.

[FIG. 1]

lysine granule

lysine core

lysine
hydrate shell

[FIG. 2]

Start

[First step] preparing lysine in granular form — S100

[Second step] exposing lysine in granular form to constant temperature and humidity environment — S200

Finish

[FIG. 3a]

[FIG. 3b]

[FIG. 3c]

[FIG. 4a]

[FIG. 4b]

[FIG. 4c]

## INTERNATIONAL SEARCH REPORT

| | International application No. |
|---|---|
| | **PCT/KR2024/004690** |

**A. CLASSIFICATION OF SUBJECT MATTER**

**A23K 20/142**(2016.01)i; **A23K 40/30**(2016.01)i; **A23K 40/10**(2016.01)i; **A23K 10/12**(2016.01)i; **C12P 13/08**(2006.01)i

According to International Patent Classification (IPC) or to both national classification and IPC

**B. FIELDS SEARCHED**

Minimum documentation searched (classification system followed by classification symbols)

A23K 20/142(2016.01); A23K 1/16(2006.01); A23K 1/175(2006.01); A23K 1/18(2006.01); A23K 10/12(2016.01); A23K 40/10(2016.01); A61K 31/198(2006.01); A61K 9/16(2006.01); C07C 229/00(2006.01); C12P 13/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 라이신(lysine), 과립(granule), 수화물(hydrate), 코어-쉘(core-shell), 유리염기(free base), 항온항습기(constants temp & humidity), 습도(humidity), 발효액(broth, fermented liquid)

**C. DOCUMENTS CONSIDERED TO BE RELEVANT**

| Category* | Citation of document, with indication, where appropriate, of the relevant passages | Relevant to claim No. |
|---|---|---|
| X | US 2009-0087518 A1 (MOORE, Kevin et al.) 02 April 2009 (2009-04-02)<br>See abstract; figures 1-14; paragraphs [0004]-[0009] and [0068]; and claims 1-31. | 1-8 |
| A | KR 10-2016-0075671 A (EVONIK DEGUSSA GMBH) 29 June 2016 (2016-06-29)<br>See claims 2-3 and 14. | 1-8 |
| A | US 2009-0017507 A1 (KUSHIKI, Takeshi et al.) 15 January 2009 (2009-01-15)<br>See paragraph [0014]. | 1-8 |
| A | US 5990350 A (STEVENS, Joseph Michael et al.) 23 November 1999 (1999-11-23)<br>See paragraph [0001], lines 22-27; and claim 20. | 1-8 |
| A | KR 10-2022-0017450 A (CJ CHEILJEDANG CORPORATION) 11 February 2022 (2022-02-11)<br>See paragraph [0021]; and claim 1. | 1-8 |

✓ Further documents are listed in the continuation of Box C.    ✓ See patent family annex.

| | |
|---|---|
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **08 August 2024** | **08 August 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/004690** |

| C. | DOCUMENTS CONSIDERED TO BE RELEVANT | |
| --- | --- | --- |
| Category* | Citation of document, with indication, where appropriate, of the relevant passages | Relevant to claim No. |
| A | KR 10-2005-0097678 A (CJ CORPORATION) 10 October 2005 (2005-10-10)<br>See claim 1. | 1-8 |

| INTERNATIONAL SEARCH REPORT | International application No. |
|---|---|
| Information on patent family members | **PCT/KR2024/004690** |

| Patent document cited in search report | | | Publication date (day/month/year) | Patent family member(s) | | | Publication date (day/month/year) |
|---|---|---|---|---|---|---|---|
| US | 2009-0087518 | A1 | 02 April 2009 | BR | PI0817701 | A2 | 07 October 2014 |
| | | | | CN | 101861098 | A | 13 October 2010 |
| | | | | CN | 101861098 | B | 19 April 2017 |
| | | | | EP | 2197295 | A1 | 23 June 2010 |
| | | | | EP | 2197295 | A4 | 10 November 2010 |
| | | | | JP | 2010-539939 | A | 24 December 2010 |
| | | | | US | 09481609 | B2 | 01 November 2016 |
| | | | | US | 2013-0305793 | A1 | 21 November 2013 |
| | | | | WO | 2009-042779 | A1 | 02 April 2009 |
| KR | 10-2016-0075671 | A | 29 June 2016 | CN | 105764348 | A | 13 July 2016 |
| | | | | CN | 105764348 | B | 02 October 2020 |
| | | | | EP | 2865274 | A1 | 29 April 2015 |
| | | | | EP | 2865274 | B1 | 04 March 2020 |
| | | | | US | 11076616 | B2 | 03 August 2021 |
| | | | | US | 2016-0255863 | A1 | 08 September 2016 |
| | | | | WO | 2015-058949 | A1 | 30 April 2015 |
| US | 2009-0017507 | A1 | 15 January 2009 | CN | 1287679 | C | 06 December 2006 |
| | | | | CN | 1437865 | A | 27 August 2003 |
| | | | | DE | 60318133 | T2 | 10 July 2008 |
| | | | | DE | 60318133 | T3 | 09 November 2017 |
| | | | | EP | 1331220 | A2 | 30 July 2003 |
| | | | | EP | 1331220 | A3 | 17 September 2003 |
| | | | | EP | 1331220 | B1 | 19 December 2007 |
| | | | | EP | 1331220 | B2 | 27 September 2017 |
| | | | | JP | 2003-219807 | A | 05 August 2003 |
| | | | | US | 2003-0152633 | A1 | 14 August 2003 |
| | | | | US | 7416740 | B2 | 26 August 2008 |
| | | | | US | 7883878 | B2 | 08 February 2011 |
| US | 5990350 | A | 23 November 1999 | CN | 1269980 | A | 18 October 2000 |
| | | | | EP | 0923878 | A2 | 23 June 1999 |
| | | | | EP | 0923878 | A3 | 15 December 1999 |
| | | | | EP | 0923878 | B1 | 14 July 2004 |
| | | | | EP | 1118673 | A1 | 25 July 2001 |
| | | | | EP | 1118673 | B1 | 27 September 2006 |
| | | | | EP | 1424012 | A2 | 02 June 2004 |
| | | | | EP | 1424012 | A3 | 09 June 2004 |
| | | | | EP | 1424012 | B1 | 11 December 2013 |
| | | | | EP | 1752543 | A1 | 14 February 2007 |
| | | | | JP | 11-253110 | A | 21 September 1999 |
| | | | | KR | 10-1999-0063252 | A | 26 July 1999 |
| | | | | TW | 521996 | A | 01 March 2003 |
| | | | | US | 06017555 | A | 25 January 2000 |
| | | | | US | 6756510 | B1 | 29 June 2004 |
| KR | 10-2022-0017450 | A | 11 February 2022 | CN | 112135607 | A | 25 December 2020 |
| | | | | CN | 112135607 | B | 04 August 2023 |
| | | | | EP | 3756654 | A1 | 30 December 2020 |
| | | | | KR | 10-2019-0111835 | A | 02 October 2019 |
| | | | | KR | 10-2021-0025574 | A | 09 March 2021 |
| | | | | KR | 10-2223797 | B1 | 12 March 2021 |
| | | | | US | 11370746 | B2 | 28 June 2022 |

Form PCT/ISA/210 (patent family annex) (July 2022)

**INTERNATIONAL SEARCH REPORT**
Information on patent family members

International application No.

**PCT/KR2024/004690**

| Patent document cited in search report | | Publication date (day/month/year) | Patent family member(s) | | | Publication date (day/month/year) |
|---|---|---|---|---|---|---|
| | | | US | 2021-0094903 | A1 | 01 April 2021 |
| | | | WO | 2019-182413 | A1 | 26 September 2019 |
| KR 10-2005-0097678 | A | 10 October 2005 | CN | 1676018 | A | 05 October 2005 |
| | | | CN | 1676018 | B | 05 May 2010 |
| | | | EP | 1582101 | A1 | 05 October 2005 |
| | | | EP | 1582101 | B1 | 07 January 2009 |
| | | | KR | 10-1052573 | B1 | 29 July 2011 |
| | | | US | 2005-0220933 | A1 | 06 October 2005 |
| | | | US | 7514111 | B2 | 07 April 2009 |
| | | | WO | 2006-004292 | A1 | 12 January 2006 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11104925 B2